# EUROPEAN PATENT SPECIFICATION

(11) **EP 0 799 174 B1**
(45) Date of publication and mention of the grant of the patent: **18.08.1999**
(21) Application number: 95942717.0
(22) Date of filing: 20.12.1995
(51) Int. Cl.: C07C 33/20, C07C 33/46, C07C 33/30, A61K 31/045

(54) **BIOCIDAL ALCOHOLS, THEIR PRODUCTION AND THEIR USE**
BIOZIDE ALKOHOLE, IHRE HERSTELLUNG UND IHRE VERWENDUNG
ALCOOLS BIOCIDES, LEUR PRODUCTION ET LEUR UTILISATION

(30) Priority: 21.12.1994 DE 4447361
(43) Date of publication of application: 08.10.1997
(73) Proprietor: SCHÜLKE & MAYR GMBH, 22851 Norderstedt (DE)
(72) Inventor: BERSCHEID, Ralf, D-22359 Hamburg (DE); EGGENSPERGER, Heinz, D-22397 Hamburg (DE); BEILFUSS, Wolfgang, D-22339 Hamburg (DE); BEHRENDS, Sabine, D-25421 Pinneberg (DE); PUCHSTEIN, Burghard, D-22309 Hamburg (DE)
(74) Representative: UEXKÜLL & STOLBERG
(86) International application number: EP9505068
(87) International publication number: WO9619428

(56) References cited:
- US-A- 4 110 430
- US-A- 4 115 578
- US-A- 4 691 043
- US-A- 4 720 581
- TETRAHEDRON, vol. 50, no. 25, 20 June 1994, OXFORD, GB, pages 7343-7366, XP002000718 D.P. CURRAN, ET AL.: "Amide-based protecting/radical translocating (PRT) groups. Generation of radicals adjacent to carbonyls by 1,5-hydrogen transfer reactions of o-iodoanilides"
- JOURNAL OF ORGANOMETALLIC CHEMISTRY, vol. 168, no. 1, 13 March 1979, LAUSANNE, CH, pages 1-11, XP002000719 J.G. DUBOUDIN, ET AL.: "Réactifs de Grignard vinyliques gamma fonctionnels. I. Réactivité des organomagnésiens vis-à-vis d'alcools alpha acétyléniques en présence d'halogénures cuivreux"
- JOURNAL OF THE AMERICAN CHEMICAL SOCIETY, vol. 53, no. 7, July 1931, WASHINGTON, DC, US, pages 2747-2755, XP002000720 M.T. BOGERT, ET AL.: "Researches on aldehydes. IV. The catalytic reduction of simple and of substituted cinnamic aldehydes"
- JOURNAL OF THE AMERICAN CHEMICAL SOCIETY, vol. 53, no. 4, April 1931, WASHINGTON, DC, US, pages 1605-1609, XP002000721 M.T. BOGERT, ET AL.: "The synthesis of simple and of substituted 2-alkylcinnamic alcohols, including a monomolecular cubebin"
- JOURNAL OF THE AMERICAN CHEMICAL SOCIETY, vol. 88, no. 24, 20 December 1966, WASHINGTON, DC, US, pages 5809-5816, XP002000722 J.R. OWEN, ET AL.: "The migration aptitude of substituted benzyl vs. methyl in carbonium ion reactions of the 2,2-dimethyl-3-aryl-1-propyl system. The question of alkyl participation"
- BERICHTE DER DEUTSCHEN CHEMISCHEN GESELLSCHAFT, vol. 67, 1934, WEINHEIM, DE, pages 1696-1712, XP002000742 J. VON BRAUN, ET AL.: "Die Unstzung von Aldehyden mit Metallen und ihre katalytische Druck-Hydrierung"
- BULLETIN DE LA SOCIETE CHIMIQUE DE FRANCE, DEUXIEME PARTIE - CHIMIE ORGANIQUE, BIOCHIMIE., no. 7-8, July 1974 - August 1974, PARIS, FR, pages 1607-1613, XP002000723 A. CARD, ET AL.: "Recherches dans la série des métallocènes. XXIX. Synthèse de benchrotrénocyclohexénones substituées par un reste isopropyle"

## Description

The invention relates to biocidal alcohols, their production and their use. In particular, the invention relates to a group of antimicrobially, fungicidally and antimycobacterially effective alcohols, to a process for their production and to the use of these alcohols in disinfectants, antiseptics, antimycotics, deodorants and preservatives.

The antimicrobial action of aliphatic alcohols is sufficiently known. Their disinfecting action increases with increasing chain length and reaches an optimum, say, in the case of 1-octanol. Primary alcohols are generally more effective than the corresponding secondary alcohols, and these in turn surpass the action of the corresponding tertiary alcohols, i.e. the action decreases e.g. in the order n-butanol - sec. butanol - tert. butanol.

2-ethyl hexanol has proved particularly effective. Unfortunately, however, this alcohol has an intensive and unpleasant odour which cannot be masked in practice by adding various perfumes. Its use as an active ingredient in disinfectants or preservatives is therefore severely limited.

The alcohols usually used, ethanol, isopropanol and n-propanol usually have to be used in concentrations of more than 50 % by wt. for the disinfection of surfaces. To deactivate viruses which are important as regards hygiene - such as e.g. Hepatitis B - the alcohol contents of hand disinfectants have to be increased to above 80 % by wt.

Disinfectants with high alcohol contents have a series of disadvantages such as for example low flash points, inadequate material compatibility above all with plastics such as e.g. plexiglas, a rapid evaporation from the skin and surface areas to be disinfected and thus no sufficient long term action, such as is e.g. indispensable for surgical hand disinfection, and an incompatibility with mucous membranes and wounds; concentrations of above 10 % by wt. already lead to an unpleasant burning.

From the series of alkyl aryl alcohols, benzyl alcohol, phenethyl alcohol and 3-phenyl-1-propanol are known to be antimicrobially effective. Benzyl alcohol is relatively easily oxidized to benzaldehyde which draws attention to itself in practice by its smell of bitter almonds. Phenethyl alcohol is the main constituent of rose oil and determines the character of the odour particularly when used for preserving cosmetics. Because of their weak action against fungi, both benzyl alcohol and phenethyl alcohol have to be combined with other active ingredients. 3-phenyl-1-propanol definitely presents itself as an antimicrobial active ingredient because of its pleasant and mild odour; however, its antimicrobial action, is unfortunately not sufficient for it to be used by itself as a disinfectant or preservative.

Also known is the antimicrobial action of the phenoxyalkanols, e.g. phenoxyethanol or 2-phenoxy-1-propanol. It is also used in practice for preserving cosmetics. The effectiveness - particularly against fungi - does however demand a relatively high use concentration. These alcohols have therefore to be combined with other active ingredients, e.g. with cationic compounds and/or aldehydes, particularly for the production of disinfectants.

In Tetrahedron, 50, 7343-66, 1994, J. Organometallic. Chem., 168, 1-11, 1979, J. Am. Chem. Soc., 53, 2747-2755, 1931, J. Am. Chem. Soc., 53, 1605-1609, 1931, J. Am. Chem. Soc., 88, 5809-5816, 1966, Berichte, 67, 1696-1712, US-A-4 li5 578, US-A-4 110 430 are described alcohol or formulae I and II. However, the alcolhols are not comprised by the subject matter of this invention. Alkyl aryl alcohols such as benzyl alcohol, phenetyl alcohol and 3-phenylpropanol are known to be antimicrobially effective. Due to their unpleasant odour or their weak antimicrobial action their use it limited. The cited documents do not suggest a solution to overcome the unpleasant odour and maintain or increase the biocidal activity.

It is therefore the object of the invention to find especially antimicrobially and fungicidally effective alcohols which, used alone or in combination with the aforementioned alcohols, produce disinfectants or preservatives which are characterized by a reduced total alcohol content, an excellent action against microorganisms - preferably against fungi - and an acceptable odour.

To achieve this object, the novel compounds (alcohols) of general formulae I and II are proposed according to claim 1: 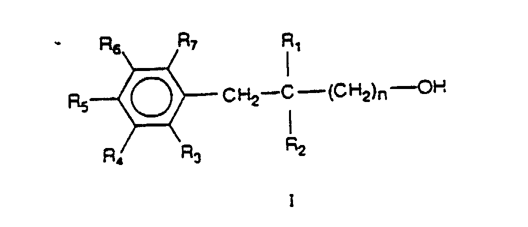 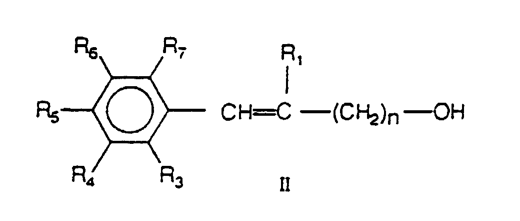 in which
- R₂: is selected from C₁-C₈ alkyl, uninterrupted or interrupted by oxygen and/or sulphur atoms, C₂-C₈ alkenyl and C₃-C₈ alkynyl,
- R₁: is a significance of R₂, independently of R₂, or in compounds of formula I is hydrogen,
each of R₃ to R₇, independently, is a significance of R₂, optionally attached to the aromatic ring by -S- or -O-, is H, halogen, nitrile or thiocyanate, and
- n: is 1 or 2,
with the proviso, that in compounds of formula I
i) where R₁ and all groups R₃ to R₇ are hydrogen, then n = 2;
ii) where R₁ and R₂ are C₁-C₆ alkyl and a) all groups R₃ to R₇ are hydrogen or b) R₅ is methyl, methoxy or chloride and all other groups R₃, R₄, R₆ and R₇ are hydrogen, then n =2;
iii) where R₁, R₂ and R₄ are methyl and all groups R₃ and R₅ to R₇ are hydrogen, then n =2;
iv) where R₁ and all groups R₃, R₄, R₆ and R₇ are hydrogen and R₅ is methyl, isopropyl, tert. butyl or methoxy, then n = 2;
v) where R₁, R₃, R₆ and R₇ are hydrogen, R₂ is methyl and R₄ and/or R₅ are H or C₁-C₆ alkyl, then n = 2;
vi) where R₁ and R₄ to R₇ are hydrogen, R₂ is methyl or ethyl and R₃ is methyl or methoxy, then n = 2;
vii) where R₁, R₃, R₅ and R₇ are hydrogen, R₂ is methyl, R₄ and R₆ are methyl or R₄ is hydrogen and R₆ is methyl, then n = 2;
viii) where R₁ is hydrogen, R₂ is butyl, R₃ and R₅ are chloride and all other groups R₄, R₆ and R₇ are hydrogen, then n = 2;
and with the proviso, that in compounds of formula II
ix) where R₁ is C₁ - C₅ alkyl or allyl and all other groups R₃ to R₇ are hydrogen, then n = 2, and
x) where R₁ is methyl, R₅ is methyl and all other groups R₃, R₄, R₆ and R₇ are hydrogen, then n = 2.

These alcohols can be produced in accordance with the process according to Claim 10 or 11.

Preferred embodiments are the subject-matter of the dependent claims.

It has surprisingly been shown that the action of the parent compound of the alcohols according to the invention, i.e. 3-phenyl-1-propanol or 4-phenyl-1-butanol or the corresponding propenols or butenols, in particular against fungi, is significantly increased when substituents are introduced into the 2-position in the case of the propanols, i.e. n = 1, or into the 3-position in the case of the butanols, i.e. n = 2, and optionally additionally into the aromatic core.

In preferred embodiments
- R₂: is selected from C₁-C₅ alkyl, uninterrupted or interrupted by oxygen and/or sulphur atoms, C₂-C₅ alkenyl and C₃-C₅ alkynyl,
- R₁: is a significance of R₂, independently of R₂, or in compounds of formula I is hydrogen,
each of R₃ to R₇, independently, is a significance of R₂, optionally attached to the aromatic ring by -S- or -O-, is hydrogen, fluorine, chlorine or bromine,
and preferably
R₂ is methyl ethyl, ethenyl, propyl, propenyl, propargyl, butyl and amyl,
R₁ is a significance of R₂, independently of R₂, or in compounds of formula I is hydrogen,
each of R₃ to R₇, independently, is a significance of R₂, is hydrogen, methyl-X-, ethyl-X-, ethenyl-X-, propyl-X-, propenyl-X-, propargyl-X, isopropyl-X, isopropenyl-X-, t-butyl-X-, methoxymethyl-X-, methoxyethyl-X-, ethoxymethyl-X-, ethoxyethyl-X-, methoxypropyl-X- or ethoxypropyl-X-, where X is -O- or -S-.

It is preferred that n = 1.

Any combinations of groups according to the above definitions are also possible.

These alcohols according to the invention are suitable as antimicrobial and fungicidal active ingredients for disinfectants, antiseptics, antimycotics, deodorants and preservatives.

The invention covers also a composition which contains at least one of said compounds of formula I or II and a compound selected from alcohols, surfactants and solvents. It is preferred that the composition contains a compound of formula I or II in a quantity of 0.01 to 10 % by wt., in particular 0.05 to 8 % by wt. and preferably 0.1 to 5 % by wt. More preferred a composition according to the invention contains
a) 0.01 to 10 % by wt. of a compound of formula I or II, and
b) 0.1 to 90 % by wt. of a compound selected from C₁-C₆ alkyl alcohols, unsubstituded or substituted with a C₆-C₁₂ aryl, aralkyl or aryloxy group, anionic, cationic, amphoteric or nonionic surfactants, dimethylformamide, betaines and glycerine.

Preferred compounds summarized in b) are, for example, ethyleneglycol ethers such as "Rewopal MPG 40" (which is tetraethyleneglycol monophenyl ether), ethoxylated higher alkyl alcohols such as "Brij 58" (which is polyoxyethylene-20-cetylalcohol), ethanol, 1-propanol, 2-propanol sulfosuccinate, betaine, phenoxyethanol and phenethylalcohol.

Said alkyl alcohols or mixtures thereof may be present in an amount of 20 to 85 % by wt., specifically 25 to 80 % by wt. Said surfactants or mixtures thereof may be present in an amount of 1 to 30 % by wt., specifically 5 to 25 % by wt. The other mentioned compounds may each be present in an amount of 0.1 to 20 % by wt., specifically 0.5 to 20 % by wt, e.g. 1.0, 2.0 or 3.0 and up to 10 or 12 % by wt.

The invention also covers the production of said compounds of formula I or II. Described in DE 35 31 585 is the production of such alcohols-using Grignard reactions. However, the disadvantages of Grignard reactions are adequately known.

The process according to the invention offers several advantages over the Grignard processes. It is particularly advantageous that according to the invention all alcohols of general formula I can be produced according to the same process. This is a malonic ester synthesis with subsequent decarboxylation and reduction. In the case of n = 2, the alcohols of general formula I can be obtained via the compounds of formula II using alkylation instead of hydrogenation.

This uniform and simple process consists of the following reaction steps: 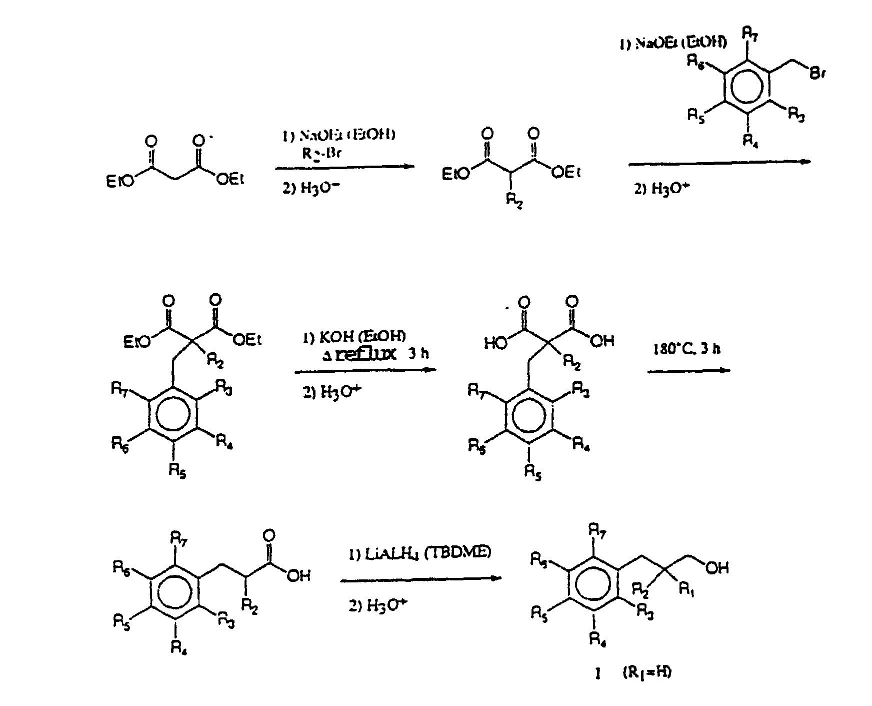
1. Alkylation of dialkyl malonate, preferably diethyl malonate with an alkyl halide, preferably a bromide, to give the monosubstituted malonic ester, as a result of which the group R₂ is introduced.
2. Second alkylation with an aryl-substituted benzyl halide, preferably a chloride or bromide, as a result of which the groups R₃ to R₇ are introduced, provided they are not hydrogen.
3. Saponification and subsequent decarboxylation to give the 3-aryl-substituted propionic acid and treatment by distillation of same.
4. Reduction to the desired alcohol of formula I, e.g. with lithium aluminium hydride in diethyl ether or tert.-butyl methylether.

The alcohols of formula II with n = 1 can for example be obtained via a Perkin condensation reaction of a corresponding aromatic aldehyde with anhydrides with simultaneous decarboxylation and subsequent reduction of the acid in question with lithium aluminium hydride.

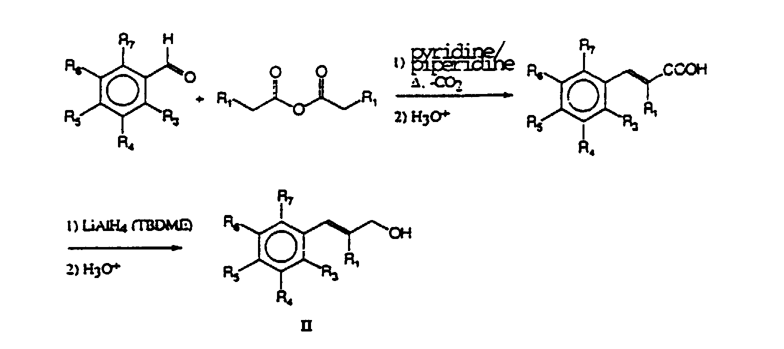

The alcohols of formula II with n = 2 are obtained for example from the respective alcohols with n = 1 via a chain elongation. The tosylate of alcohol II (n = 1) is substituted nucleophilically by NaCN and saponified. The resulting acid can be reduced with lithium aluminium hydride to the desired alcohol II (n=2).

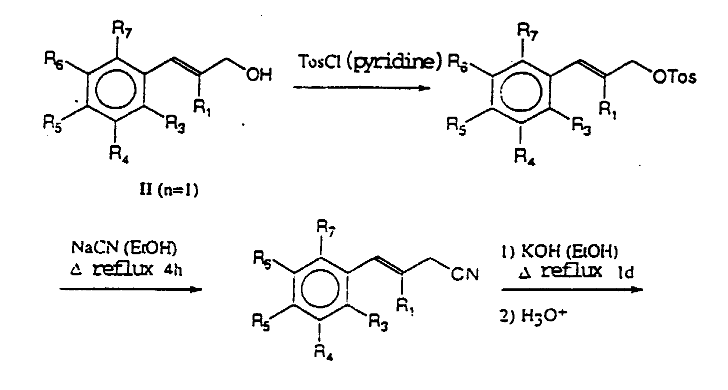

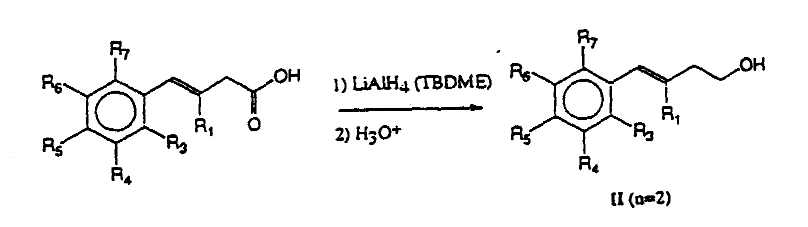

The alcohols I with n = 2 can be obtained in analogous manner.

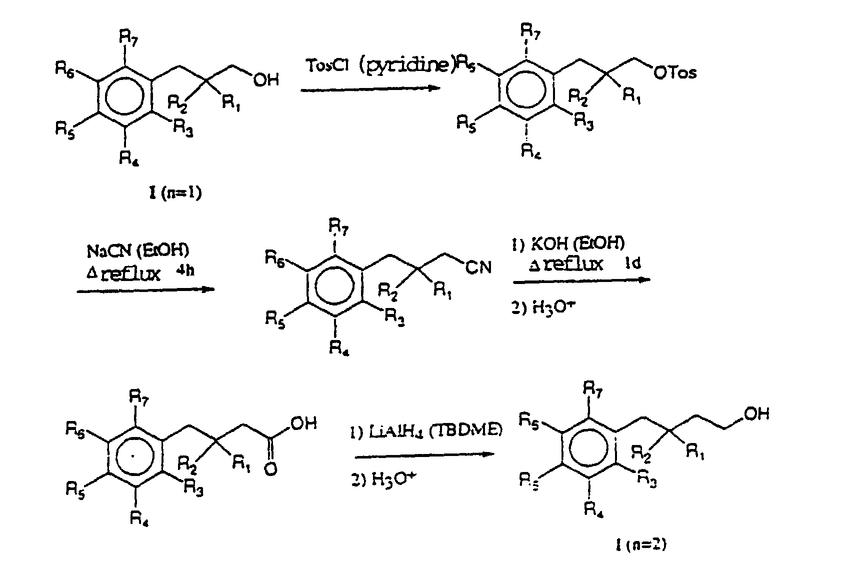

By reducing alcohols of formula II with a reducing agent such as lithium aluminium hydride or alkylation agents such as lithium dialkyl cuprate or trialkyl boron, the alcohols of formula I can be obtained.

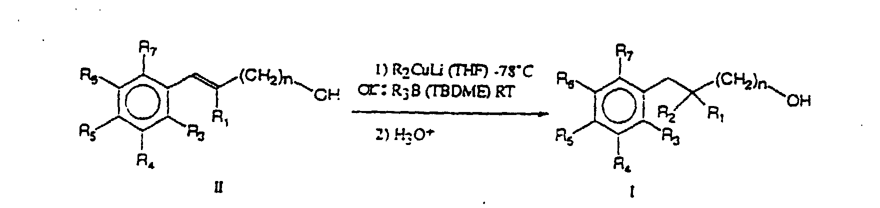

### General synthesis instructions for alcohols of formula I using malonic acid diethyl ester

### 1. General instructions for the first alkylation of malonic acid diethyl esters:

200 mmol malonic acid diethyl ester and 200 mmol R₂-alkyl bromide (or chloride) are introduced first into a 250 ml triple-necked flask with internal thermometer, reflux condenser and dropping funnel and the whole is cooled to 10 to 15°C. 68.05 g (200 mmol) 20 % NaOEt in EtOH are slowly added dropwise (over 30 minutes) via a dropping funnel so that the temperature does not exceed 20°C. The mixture is then stirred for a further 30 minutes at 20°C and finally heated to 50 to 60°C for 1 hour. After cooling, the mixture is neutralized with glacial acetic acid (optionally cooling; pH monitored until the buffer pH value is reached). The resulting NaBr is separated off with a frit and then washed with a little cold EtOH. The main quantity of alcohol in the filtrate is distilled off at normal pressure. The filtrate is mixed with 50 ml H₂O and 1 ml conc. HCl, and the organic and the aqueous phases are separated from one another. The organic phase is kept for further use (see below) and the aqueous phase is extracted with 2 x 50 ml ether (if phase separation does not take place, the filtered-off NaBr is used to increase the density, as a result of which a phase separation is initiated). The combined organic phases are dried over sodium sulphate and the solvent is removed in a vacuum. The thus-formed crude product (R₂-substituted malonic ester) can be further used directly for the subsequent saponification.

### 2. General instructions for the second alkylation of alkyl malonic acid diethyl esters:

200 mmol R₂-substituted malonic acid diethyl ester and 200 mmol R₃-R₇-substituted benzyl bromide (or chloride) are introduced first into a 250 ml triple-necked flask with internal thermometer, reflux condenser and dropping funnel and the whole is cooled to 10 to 15°C. 68.05 g (200 mmol) of 20 % NaOEt in EtOH are slowly added dropwise (over 30 minutes) via a dropping funnel so that the temperature does not exceed 20°C. The mixture is then stirred for a further 30 minutes at 20°C and finally heated to 50 to 60°C for 1 hour. After cooling, the mixture is neutralized with glacial acetic acid (optionally cooling; pH monitored until the buffer pH value is reached). The resulting NaBr is separated off with a frit and then washed with a little cold EtOH. The main quantity of alcohol in the filtrate is distilled off at normal pressure. The filtrate is mixed with 50 ml H₂O and 1 ml conc. HCl, and the organic and the aqueous phases are separated from one another. The organic phase is kept for further use (see below) and the aqueous phase is extracted with 2 x 50 ml ether (if phase separation does not take place, the filtered-off NaBr is used to increase the density, as a result of which a phase separation is initiated). The combined organic phases are dried over sodium sulphate and the solvent is removed in a vacuum. The thus-formed crude product (disubstituted malonic ester) can be further used directly for the subsequent saponification.

### 3. General instructions for the saponification of disubstituted malonic esters:

100 mmol of the disubstituted malonic ester are refluxed with a solution of 45 g conc. KOH (45%) and 50 ml EtOH for 3 hours. The main quantity of ethanol is distilled off under weak vacuum, the remaining residue is dissolved in H₂O until the water is clear and conc. HCl is added dropwise, accompanied by cooling with ice, until the pH value is 1. The aqueous phase is extracted with 100 ml and then 2 x 50 ml ether. The combined organic phases are dried over sodium sulphate, the solvent is removed in a vacuum and the remaining oil is dried over night in a desiccator. The crude product (disubstituted malonic acid) can be further used for the subsequent decarboxylation without further purification; small residual quantities of ethanol or water do not cause disturbance.

### 4. General instructions for the decarboxylation of disubstituted malonic acids:

The disubstituted malonic acid is heated for 3 hours at 180°C (CO₂ cleavage). Residual quantities of ethanol and H₂O and fruit esters are then distilled off at normal pressure (bath temperature 230 to 250°C). After applying a vacuum (20 to 25 mbar) the 2,3-disubstituted propionic acid is subjected to fractional distillation. To remove moisture that has distilled over and not very volatile components, the distillates can be dried in a desiccator.

### 5. General instructions for reducing disubstituted propionic acids with lithium aluminium hydride:

3.13 g (82.5 mmol) LiAIH₄ are introduced first into 100 ml of abs. ether. 100 mmol 2,3-disubstituted propionic acid in 50 ml ether are then slowly added dropwise (possibly with cooling), so that the ether boils easily. After the addition is finished, the mixture is stirred for a further 1 h at room temperature and then refluxed for 4 h. The cooled reaction mixture is carefully introduced with stirring into 200 ml iced water and stirred until the evolution of hydrogen is no longer to be observed. The whole is then mixed with 50 ml 10 % H₂SO₄, as a result of which the aluminium hydroxide precipitate dissolves. The phases are separated and the aqueous phase is extracted with 3 x 100 ml ether. The combined organic phases are washed with 3 x 50 ml of semi-concentrated NaOH and 2 x 50 ml saturated NaCl solution, dried over sodium sulphate and the solvent is removed in vacuum. The 2,3-disubstituted propanol is purified by distillation.

### Synthesis examples

Selected as synthesis examples were
(±)-2-benzyl butanol (R₁=H; R₂=Et; R₃=R₄=R;=R₆=R₇=H),
(±)-2-(3-methylbenzyl) butanol (R₁=H, R₂=Et; R₃=H; R₄=Et; R₅=R₆=R₇=H)
and
(±)-2-(3-chlorobenzyl) butanol (R₁=H, R₂=Et; R₃=H; R₄=Cl; R₅=R₆=R₇=H).

### (±)-2-benzyl butanol:

20 % total yield; colourless liquid with weak, pleasant odour; d = 0.975; n_{D}20 = 1.5178; IR corresponds to the structure. 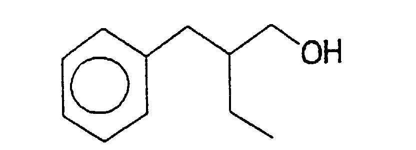 ¹H-NMR: 0.90 (t; 3H, CH₂C*H*₃), 1.30 (dq; 2H, C*H*₂CH₃), approx. 1.65 (m; 1H, C*H*), 2.30 (s; 1H, O*H*), 2.60 (d; 2H, ArC*H*₂), 3.45 (d; 2H, C*H*₂OH), 7.0-7.4 ("s"; 5H, Ar*H*).

### (±)-2-(3-methylbenzyl) butanol:

16 % total yield; colourless liquid with slight lily of the valley-type odour; d = 0.963; n_{D}20 = 1.5152; IR corresponds to the structure. 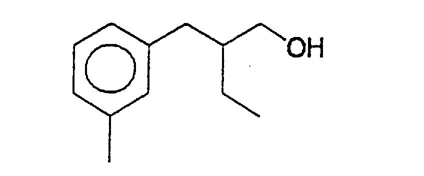 ¹H-NMR: 0.90 (t; 3H, CH₂C*H*₃), 1.30 (dq; 2H, C*H*₂CH₃), approx. 1.6 (m; 1H, C*H*), 2.25 (s; 3H, ArC*H*₃), 2.40 (s; 1H, O*H*), 2.55 (d; 2H, ArC*H*₂), 3.45 (d; 2H, C*H*₂OH ), 6.7-7.2 (m; 4H, Ar*H*).

### (±)-2-(3-chlorobenzyl) butanol:

16 % total yield; slightly yellow liquid with discreet, pleasant odour; d = 1.099; n_{D}20 = 1.5322; IR corresponds to the structure. 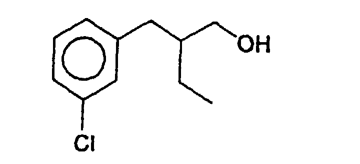 ¹H-NMR : 0.90 (t; 3H, CH₂C*H*₃), 1.30 (dq; 2H, C*H*₂CH₃), 1.55 (m; 1H, C*H*)*,* 2.55 (d; 2H, ArC*H*₂), 3.30 (s; 1H, O*H),* 3.45 (d; 2H, C*H*₂OH), 6.9-7.2 ("s"; 4H, Ar*H*).

Formulae of the alcohols treated below:

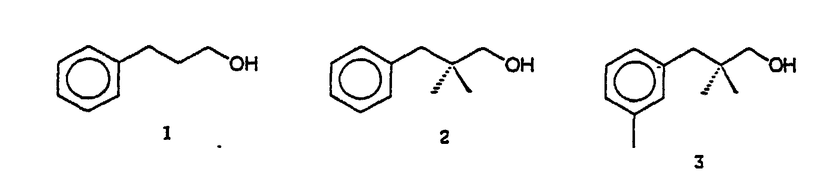

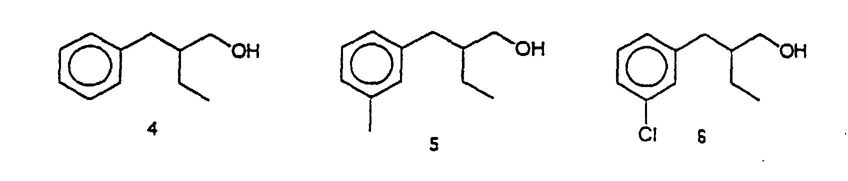

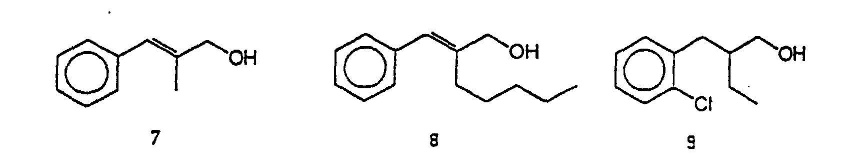

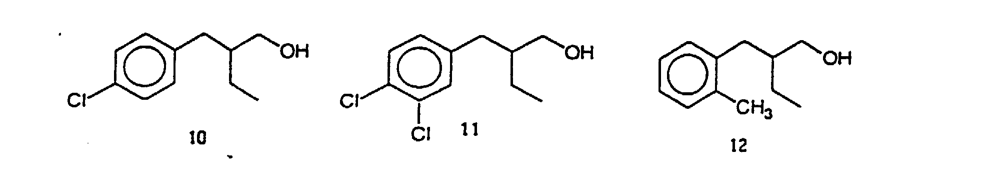

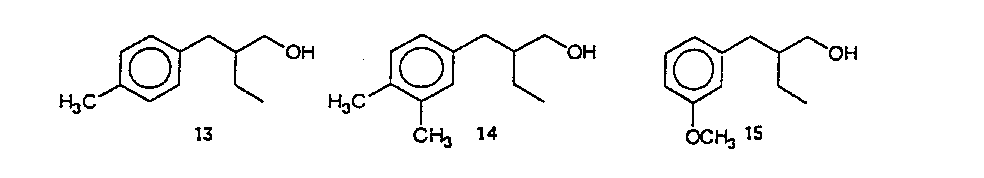

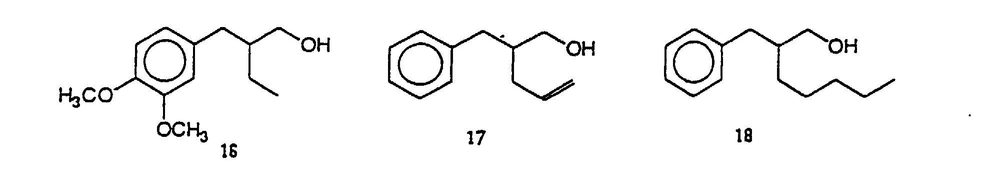

### Applications

### 1. MIC (minimum inhibiting concentration) values

### a) MIC values, water-soluble

Standard formulation:
- Rewopal MPG 40 25.0 g
- aromatic alcohol 10 mmol
- dem.* water to 100 g
- lactic acid for adjusting the pH value to 7.0 q.s.

| | | |
|---|---|---|
| Test germs | Staphylococcus aureus | ATCC 6538 |
| | Proteus vulgaris | NCTC 4635 |
| | Candida albicans | ATCC 10231 |
| | Penicillium funiculosum | ATCC 36839 |
| | Aspergillus niger | ATCC 6275 |

(*dem. = demineralized)

### Test method:

In sterile test tubes, 5 ml each of the dilutions of the disinfectant in WSH (water of standardized hardness) are mixed with 5 ml double-concentrated casein peptone soybean flour peptone solution (CSL) or CSL and deactivating substances.

To determine the bacteriostatic action on Staphylococcus aureus and Proteus mirabilis the tubes are inoculated by adding 0.1 ml of a CSL culture diluted 1:10 with CSL and incubated for 24 h at 37°C.

To test the fungistatic action, 0.1 ml of an undiluted CSL culture of Candida albicans which has been incubated at 37°C for 72 h is used in each case. Evaluation takes place after 72 h at 37°C.

The highest dilution of the preparation in CSL or CSL and deactivating substances that still suppresses growth of the test germs after 12 h incubation serves as the measure of the multiplication-inhibiting action (inhibition titre).

In the case of the disinhibition tests, the culture media are to be adjusted to a pH value of 7.0 ± 0.2 according to the state of the disinfectant.

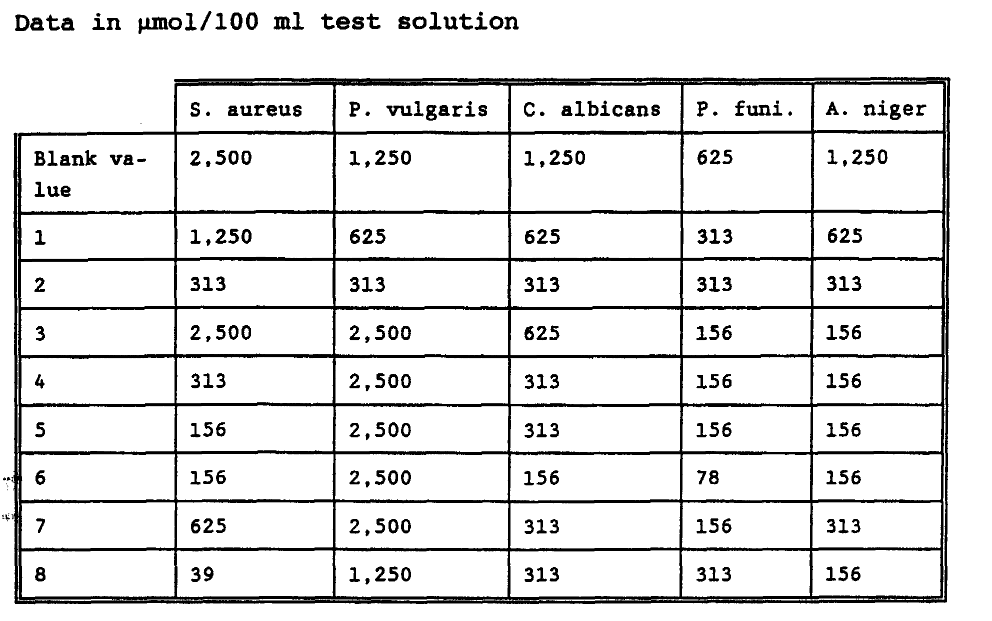

Standard formulation:
- aromatic alcohol 5.0 %
- Brij 58 5.0 %
- 1,3-butanediol to 100

Test germs: see above
Test method: see above

| Data in µmol/100 ml test solution | | | | | |
|---|---|---|---|---|---|
| Compd. No. | S. aureus | P. vulgaris | C. albicans | P. funi. | A. niger |
| Blank value | 2,500 | 1,250 | 1,250 | 625 | 1,250 |
| 1 | 1,250 | 625 | 625 | 313 | 625 |
| 3 | 625 | 625 | 625 | 313 | 625 |

Compared with the parent compound 3-phenyl propanol (alcohol Compd. No. 1), the alcohols 2-8 according to the invention clearly display microbistatic activities, particularly alcohols 2, 6 and 8, in almost ten times lower a use concentration.

### b) MIC values, water-insoluble

| Solutions of the aromatic alcohols in acetone (w/w) | | |
|---|---|---|
| Test germs | Staphylococcus aureus | ATCC 6538 |
| | Escherichia coli | ATCC 11229 |
| | Candida albicans | ATCC 10231 |
| | Aspergillus niger | ATCC 6275 |
| | | |
| Test method | as under 1.; the dilution solutions were prepared in acetone. | |

The size of the covered areas of the plates is given in %; 100% means no inhibiting action.

| Alcohol | Concentration [% by wt.] | S. aureus | E. coli | C. albicans | A. niger |
|---|---|---|---|---|---|
| Blank value | 0.00 | 100% | 100% | 100% | 100% |
| 9 | 1.00 | 80% | 100% | 80% | 20% |
| | 0.50 | 100% | 100% | 100% | 90% |
| | 0.25 | 100% | 100% | 100% | 100% |
| 10 | 1.00 | 10% | 100% | 10% | 10% |
| | 0.50 | 100% | 100% | 90% | 70% |
| | 0.25 | 100% | 100% | 100% | 90% |
| | 0.125 | 100% | 100% | 100% | 100% |
| 11 | 1.00 | 5% | 90% | 10% | 10% |
| | 0.50 | 90% | 100% | 80% | 70% |
| | 0.25 | 100% | 100% | 100% | 100% |
| 12 | 1.00 | 90% | 100% | 80% | 80% |
| | 0.50 | 100% | 100% | 100% | 100% |
| 13 | 1.00 | 90% | 95% | 90% | 20% |
| | 0.50 | 100% | 100% | 100% | 90% |
| | 0.25 | 100% | 100% | 100% | 100% |
| 14 | 1.00 | 30% | 100% | 20% | 10% |
| | 0.50 | 90% | 100% | 100% | 80% |
| | 0.25 | 100% | 100% | 100% | 90% |
| | 0.125 | 100% | 100% | 100% | 100% |
| 15 | 1.00 | 100% | 100% | 100% | 90% |
| | 0.50 | 100% | 100% | 100% | 100% |
| 17 | 1.00 | 100% | 90% | 100% | 80% |
| | 0.50 | 100% | 100% | 100% | 100% |
| 18 | 1.00 | 0% | 100% | 70% | 0% |
| | 0.50 | 20% | 100% | 80% | 40% |
| | 0.25 | 100% | 100% | 100% | 100% |

Alcohols 11 and 13 display a very good broad activity spectrum. In contrast, alcohols 10, 14 and 18 display a very good selective action, in particular against fungi and yeasts.

### 2. Antimicrobial effectiveness in the plate diffusion test

Standard formulation:
- aromatic alcohol 1 part
- dimethylformamide 6 parts

| | | |
|---|---|---|
| Test germs | Staphylococcus aureus | ATCC 6538 |
| | Pseudomonas aeruginosa | ATCC 15442 |
| | Proteus mirabilis | ATCC 14153 |
| | Escherichia coli | ATCC 11229 |
| | Candida albicans | ATCC 10231 |
| | | |
| Test method | Agar diffusion test | |

**The diameters of the inhibition zones are given in mm.**

| Alcohol | S. aureus | P. aeruginosa | P. vulgaris | E. coli | C. albicans |
|---|---|---|---|---|---|
| Blank value | 0 | 0 | 0 | 0 | 0 |
| 9 | 15 | 0 | 0 | 11 | 15 |
| 10 | 14 | 0 | 0 | 11 | 15 |
| 11 | 17 | 0 | 0 | 0 | 13 |
| 12 | 20 | 15 | 13 | 17 | 22 |
| 13 | 16 | 13 | 14 | 13 | 19 |
| 14 | 18 | 18 | 0 | 15 | 22 |
| 15 | 18 | 15 | 18 | 18 | 23 |
| 16 | 18 | 18 | 17 | 17 | 28 |
| 17 | 16 | 12 | 13 | 13 | 17 |
| 18 | 11 | 0 | 0 | 0 | 11 |

Alcohols 12, 15 and 16 show a very strong inhibition of the tested germs, alcohols 13, 14 and 17 showing a strong inhibition.

### 3. Use in an alcoholic surface disinfectant

Standard formulation:
- ethanol (MEK denatured) 25.0 %
- 1-propanol 35.0 %
- perfume 0.02 %
- benzotriazole 0.001 %
- Marlipal 013/70 0.1%
   (isotridecanpolyethyleneglycol-(7)-ether = C₁₃ oxo alcohol + 7 mol ethylene oxide)
- active ingredient additive x%
- dem. water to 100
Test germ: Ps. aeruginosa
Test method:

Quantitative surface test according to DGHM (Deutsche Gesellschaft für Hygiene and Microbiology = German Association for Hygiene and Microbiology). In order to exclude the effectiveness of the readily volatile alcohol components (ethanol, 1-propanol), the preparations were deposited onto the surfaces and the germs were deposited after approx. 20 minutes.
Test surfaces: PVC and OP tiles
Data as reduction factors (log stages)

| | PVC | | | Tiles | | |
|---|---|---|---|---|---|---|
| Additive | 30' | 60' | 240' | 30' | 60' | 240' |
| without additive | 0 | 0 | 0 | 0 | 0 | 0 |
| 0.05 % phenoxyethanol | 0 | 0 | 0 | 0 | 0 | 0 |
| 0.05% phenoxyethanol 0.01% imidazole | 0 | 0 | 0 | 0 | 0 | 0 |
| 0.125% Vantocil IB (polyhexamethylene biguanid hydrochlorid) 0.025% sorbic acid | 0 | 0 | 0 | 0 | 0 | 0 |
| 0.027% Hostapur SAS (sec.alkanesulphonate-Na-salts based on n-paraffins) 0.006% Na-laurylether sulphate 0.017% malic acid | 0 | 0 | 0 | 0 | 0 | 0 |
| 0.05% 3-phenyl propanol (1) | 0 | 0 | 0 | 0 | 0 | 0 |
| 0.05% 2,2-dimethyl-3-phenyl-1-propanol (2) | >6.0 | >5.4 | >6.5 | 4.1 | 4.9 | >5.8 |

Only the preparation with an aromatic aiconoi of formula I according to the invention, 2,2-dimethyl-3-phenyl-1-propanol (2) has an effectiveness against Pseudomonas aeruginosa on PVC and tiles that increases with increasing action time.

The other preparations are disinfectant solutions.

### 4. Use in a foot spray with deodorizing action and simultaneous prevention of athlete's foot

Formula:
- 2-propanol 40.0%
- aromatic alcohol 0.2 %
- allantoin 0.5 %
- dem. water to 100

- Test germs:: special skin fungi such as Trichophyton rubrum, Trichophyton mentagrophytes (ATCC 9533), Microsporon gypseum
- Test method:: Determination of the minimum inhibition concentration (method see under 1.) Data in %

| Alcohol | T. rubrum | T. mentagrophytes | M. gypseum |
|---|---|---|---|
| Blank value | 12.5% | 6.25% | 6.25% |
| 1 | 6.25% | 6.25% | 6.25% |
| 6 | 1.56% | 1.56% | 3.13% |
| 8 | 1.56% | 1.56% | 1.56% |

- Test germs:: special skin fungi such as Trichophyton rubrum, Trichophyton mentagrophytes, Microsporon gypseum
- Test method:: Agar diffusion test Data as millimetres inhibition zone

| Alcohol | Use concentration | T. rubrum | T. mentagrophytes | M. gypseum |
|---|---|---|---|---|
| Blank value | 100% | 0 mm | 0 mm | 0 mm |
| 1 | 100% | 0 mm | 0 mm | 0 mm |
| 6 | 100% | 12 mm | 15 mm | 13 mm |
| 8 | 100% | 23 mm | 22 mm | 19 mm |
| | 50% | 14 mm | 14 mm | 10 mm |

With typical fungi which are relevant as regards skin, the formulations with alcohols 6 and 8 according to the invention show a very good action both in the MIC test and in the agar diffusion test. The aforementioned formulations are thus suitable for use in deodorants and products for the prevention of athlete's foot.

The parent compound 3-phenyl propanol shows almost similar values as the blank value, i.e. is ineffective.

### 5. Preservative

Standard formulation:
- sulfosuccinate 12.0%
- betaine 3.0%
- aromatic alcohol 0.5%
- re-fatting agent
- skin care additives
- thickener
- dem. water to 100

- Test germs:: Germ mixture of Staphylococcus aureus, Staphylococcus epidermis, Escherichia coli, Klebsiella pneumoniae, Enterobacter gergoviae, Pseudomonas aeruginosa, Pseudomonas fluorescens, Pseudomonas putida, Aspergillus niger, Penicillium funiculosum, Candida albicans; Total germ count 10⁸-10⁹/ml.
- Test method:: weekly loading of the sample with germ suspension; smear onto CS and Sabouraud agar. See also K.-H. Diehl, P. Oltmanns, J. Ramsbotham, Seife, Öle, Fette, Wachse 118 (1992) 546.

Data expressed semi-qualitatively:
- no growth < 10² CFU/g (CFU = colony-forming units)
+ slight growth approx. 10³ CFU/g
++ moderate growth approx. 10⁴-10⁵ CFU/g
+++ heavy growth > 10⁵ CFU/g

| Alcohol | 1st week | 2nd week | 3rd week | 4th week | 5th week |
|---|---|---|---|---|---|
| Blank value | +++ | +++ | +++ | +++ | +++ |
| Phenoxyethanol | - | - | - | - | - |
| 1 | + | + | - | - | - |
| 2 | - | - | - | - | - |

Preservation with 0.5 % 2,2-dimethyl-3-phenyl propanol (2) is just as effective as that with the known preservative phenoxyethanol, but displays a more sure (more quickly acting) preservation in the first two weeks compared with the parent compound 3-phenyl propanol.

The alcohols according to the invention are thus suitable as a preserving additive in shampoos and shower gels.

### 6. Mucous membrane antiseptic

Standard formulation:
- Cocamidopropyl betaine (30%) 3.0 %
- glycerin DAB 10 (85%) 0.5%
- phenoxyethanol 1.0%
- arom. alcohol 0.5%
- dem. water to 100
- NaOH to adjust the pH value to 5.5 q.s.

- Test germs:: Pseudomonas aeruginosa ATCC 15442 Staphylococcus aureus ATCC 6538
- Test method:: Quantitative suspension test according to DGHM

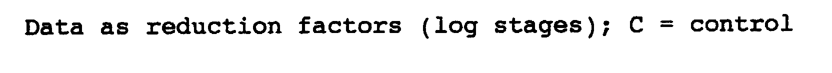

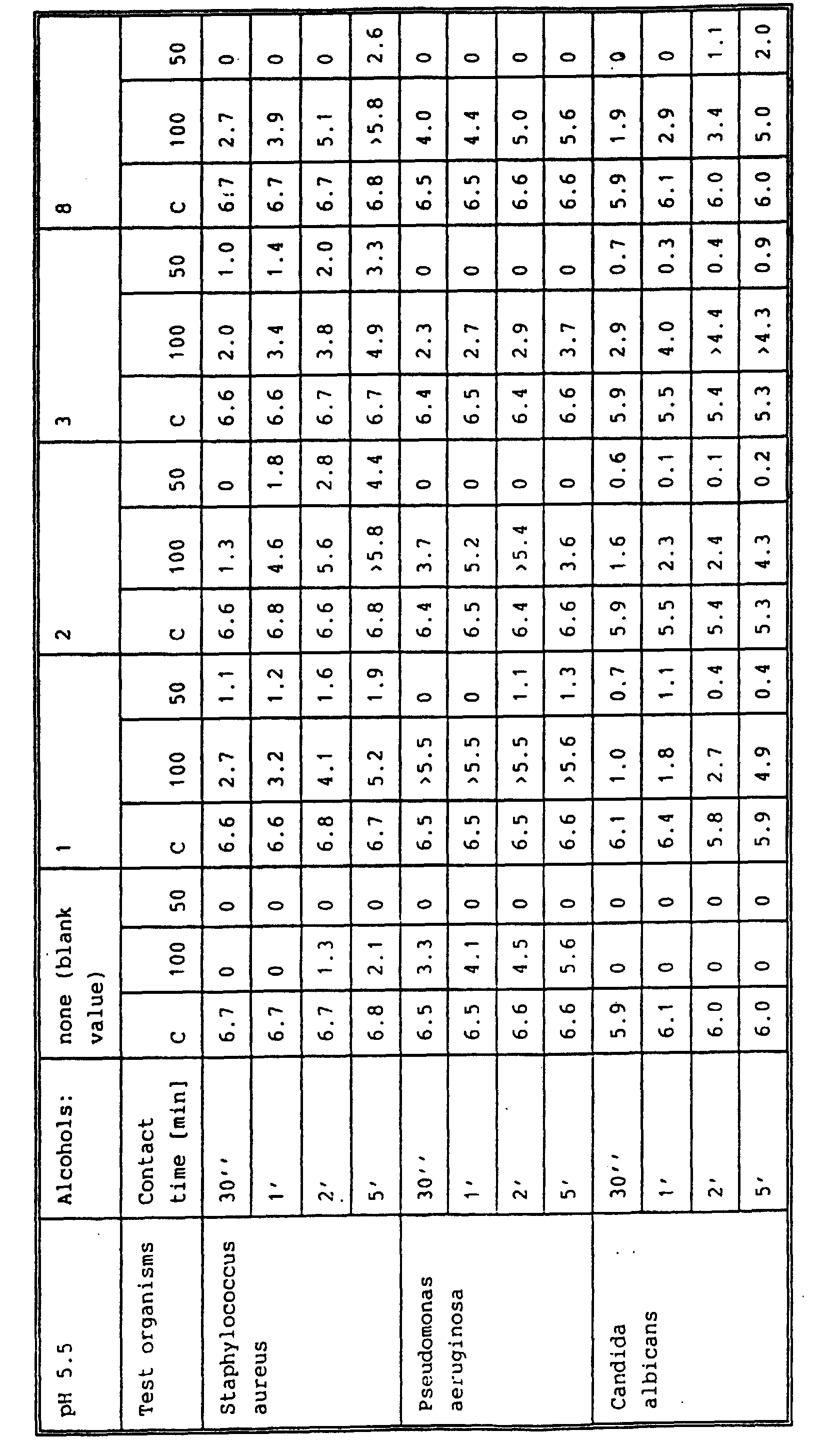

The alcohols according to the invention significantly increase the effectiveness against the aforementioned germs, in particular against yeasts.

### 7. Skin antiseptic

### a) standard formulation:

- 1-propanol 30.0%
- 2-propanol 45.0%
- aromatic alcohol 1.0%
- dem. water to 100

- Test germ:: Microsporon luteus ATCC 15442
- Test method:: Apply 0.2 ml preparation to 10cm² skin, allow to dry, cover with TEGADERM® film and leave to work for 1 h, contaminate with 0.1 ml germ suspension, remove after 15 minutes with ring method
- Reference:: Control against Neo-Kodan®
- Number of subjects:: 10 subjects

| Data as average value of the reduction factors (RF in log stages) of all 10 subjects | |
|---|---|
| aromatic alcohol | Average value of RF |
| 1.0% phenyl propanol (1) | 0 |
| 1.0% α-amyl cinnamyl alcohol (8) | 1.9 |
| Reference: Neo-Kadan® | 1.9 |

The formulation with 1.0% α-amyl cinnamyl alcohol (8) also shows the same values in the suspension test according to DGHM as the skin antiseptic Neo-Kadan® used for reference of 50%, 30 seconds, and likewise shows an equal action against the resident skin flora (100%, 15 seconds).

Moreover, the aforementioned results show that an action against the transient flora is only guaranteed when the α-amyl cinnamyl alcohol (8) substituted according to formula II is used and not the parent compound 3-phenyl propanol (1).

### b) Standard formulation:

- 1-propanol 15.0%
- 2-propanol 30.0%
- aromatic alcohol 1.0%
- dem. water to 100

| | | |
|---|---|---|
| Test germs | Staphylococcus aureus | ATCC 6538 |
| | Pseudomonas aeruginosa | ATCC 15442 |
| | Candida albicans | ATCC 10231 |
| | | |
| Test method | Quantitative suspension test according to DGHM | |

| Data as reduction factors (log stages) | | | | | | | | |
|---|---|---|---|---|---|---|---|---|
| | | Blank value (0% 8) | | | | 1.0% 8 | | |
| Test organisms | Contact time [min] | C | 75 | 50 | 25 | 75 | 50 | 25 |
| Staphylococcus aureus | 30" | 6.6 | >5.6 | >5.6 | 0 | >5.6 | >5.6 | 2.8 |
| | 1' | 6.5 | >5.5 | >5.5 | 0 | >5.5 | >5.5 | 3.6 |
| | 2' | 6.9 | >5.9 | >5.9 | 0 | >5.9 | >5.9 | 4.7 |
| | 5' | 6.8 | >5.8 | >5.8 | 0 | >5.8 | >5.8 | >5.8 |
| Pseudomonas aeruginosa | 30" | 6.6 | >5.6 | >5.6 | 0 | >5.6 | >5.6 | 0 |
| | 1' | 6.8 | >5.8 | >5.8 | 0 | >5.8 | >5.8 | 0 |
| | 2' | 6.7 | >5.7 | >5.7 | 0 | >5.7 | >5.7 | 0 |
| | 5' | 6.7 | >5.7 | >5.7 | 0 | >5.7 | >5.7 | 0 |
| Candida albicans | 30" | 5.6 | >4.6 | 0.9 | 0.2 | >4.6 | 2.7 | 0.5 |
| | 1' | 5.6 | >4.6 | 1.5 | 0 | >4.6 | 3.5 | 0.6 |
| | 2' | 5.9 | >4.9 | 2.4 | 0.4 | >4.9 | >4.9 | 1.1 |
| | 5' | 6.1 | >5.1 | 3.5 | 0 | >5.1 | >5.1 | 1.7 |

In the aforementioned propanol-reduced formulation, the additional action of the α-amyl cinnamyl alcohol is seen in particular in the case of Candida albicans.

### 8. Use in an alcoholic disinfectant for surgical hand disinfection

### Formulation:

- ethanol 80.0%
- phenethyl alcohol 2.0%
- 2,2-dimethyl-3-(3-methylphenyl) propanol (3) 0.4%
- re-fatting agent
- humectant
- dem. water to 100

The requirements of the DGHM guideline for surgical hand disinfection are satisfied by the aforementioned formula both in their immediate action and also in their long-term action.

A formulation which contains neither phenethyl alcohol nor 2,2-dimethyl-3-(3-methylphenyl) propanol (3) does not satisfy these requirements.

### 9. Effectiveness against M. terrae S in the germ carrier experiment with standard cotton

### Standard formulation:

- Rewopal MPG 40 25.0%
- aromatic alcohol 2.0 %
- dem. water to 100

| | | |
|---|---|---|
| Test germ | Mycobacterium terrae | ATCC 15755 |
| | | |
| Test method | Production of the germ carriers: To prepare the germ carriers, standard cotton fabric is used which has been thoroughly rinsed in double-distilled water. The fabric is cut into pieces measuring approximately 1 cm², sterilized in a autoclave and dried. | |

### Production of the bacterial suspension:

The bacteria are elutriated with 5 ml CSL from a 24 h-old (37°C) culture onto CSA plates measuring approx. 9 cm in diameter, the suspension being diluted with CSL if necessary. The number of CFU/ml is to be determined using surface culture. It should be > 10⁹/ml.

### Procedure for the germ carrier test:

The sterilized and dried germ carriers are introduced into the bacterial suspension and left in it for 15 minutes, during which they are turned over twice.

A number (4) of contaminated, thoroughly impregnated germ carriers, corresponding to the proposed removal times - 15, 30, 60 and 120 minutes - is placed in a small dish and 10 ml of the disinfectant solution to be tested in WSH are poured over them. Air bubbles are to be removed by repeated turning of the germ carriers.

After the corresponding action times, the germ carriers are to be removed from the disinfectant solution, and after rinsing twice in each case for 1 min in 10 ml ML solution (see Appendix) to which the deactivating substances were optionally added, the germ carriers are placed onto the surface of a Löwenstein-Jensen nutrient medium with tweezers and moved backwards and forwards 3 to 4 times using light pressure. After inoculating the nutrient medium surface the small cloth is to remain lying directly above the condensed water level of the nutrient medium.

Germ carriers pre-treated in the same way, but kept in WSH for 120 minutes instead of in disinfectant solution are to be inoculated as a control. The inoculated tubes are incubated at 37°C for 3 weeks.

| Data expressed qualitatively: | | | |
|---|---|---|---|
| E | individual colonies | ++ | moderate growth |
| M | several colonies | +++ | heavy growth |
| + | weak growth | ++++ | very heavy growth |
| ∞ | lawn growth | | |

| Alcohol | 15' | 30' | 60' | 120' |
|---|---|---|---|---|
| none | ∞ | ∞ | ∞ | ∞ |
| 1 | ++++ | ++++ | +++ | +++ |
| 2 | +++ | ++ | + | M |
| 3 | +++ | + | + | E |
| 7 | +++ | ++ | ++ | + |
| 8 | +++ | ++ | + | E |

The alcohols according to the invention, particularly 2, 3 and 8, show a very good action against mycobacteria with relatively long action times and are therefore suitable for use in instrument disinfectants. The parent compound 1 shows a very much weaker action.

## Claims

1. A compound of formula I or II, 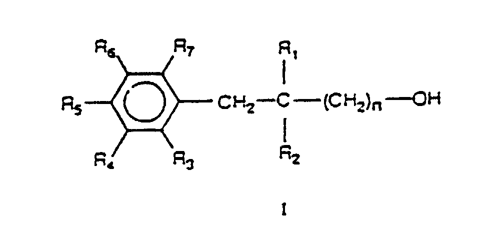 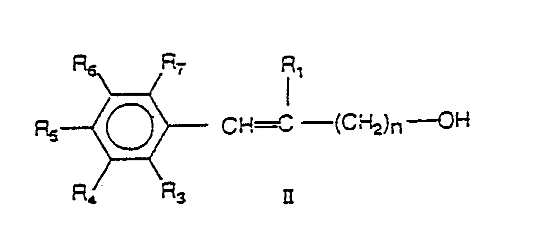 in which
R₂ is selected from C₁-C₈ alkyl, uninterrupted or interrupted by oxygen and/or sulphur atoms, C₂-C₈ alkenyl and C₃-C₈ alkynyl,
R₁ is a significance of R₂, independently of R₂, or in compounds of formula I is hydrogen,
each of R₃ to R₇, independently, is a significance of R₂, optionally attached to the aromatic ring by -S- or -O-, is H, halogen, nitrile or thiocyanate, and
n is 1 or 2,
with the proviso, that in compounds of formula I
i) where R₁ and all groups R₃ to R₇ are hydrogen, then n = 2;
ii) where R₁ and R₂ are C₁-C₆ alkyl and a) all groups R₃ to R₇ are hydrogen or b) R₅ is methyl, methoxy or chloride and all other groups R₃, R₄, R₆ and R₇ are hydrogen, then n =2;
iii) where R₁, R₂ and R₄ are methyl and all groups R₃ and R₅ to R₇ are hydrogen, then n =2;
iv) where R₁ and all groups R₃, R₄, R₆ and R₇ are hydrogen and R₅ is methyl, isopropyl, tert. butyl or methoxy, then n = 2;
v) where R₁, R₃, R₆ and R₇ are hydrogen, R₂ is methyl and R₄ and/or R₅ are H or C₁-C₆ alkyl, then n = 2;
vi) where R₁ and R₄ to R₇ are hydrogen, R₂ is methyl or ethyl and R₃ is methyl or methoxy, then n = 2;
vii) where R₁, R₃, R₅ and R₇ are hydrogen, R₂ is methyl, R₄ and R₆ are methyl or R₄ is hydrogen and R₆ is methyl, then n = 2;
viii) where R₁ is hydrogen, R₂ is butyl, R₃ and R₅ are chloride and all other groups R₄, R₆ and R₇ are hydrogen, then n = 2;
and with the proviso, that in compounds of formula II
ix) where R₁ is C₁ - C₅ alkyl or allyl and all other groups R₃ to R₇ are hydrogen, then n = 2, and
x) where R₁ is methyl, R₅ is methyl and all other groups R₃, R₄, R₆ and R₇ are hydrogen, then n = 2.

2. A compound according to claim 1,
in which
R₂ is selected from C₁-C₅ alkyl, uninterrupted or interrupted by oxygen and/or sulphur atoms, C₂-C₅ alkenyl and C₃-C₅ alkynyl,
R₁ is a significance of R₂, independently of R₂, or in compounds of formula I is hydrogen,
each of R₃ to R₇, independently, is a significance of R₂, optionally attached to the aromatic ring by -S- or -O-, is hydrogen, fluorine, chlorine or bromine.

3. A compound according to claim 1 or 2 in which
R₂ is methyl ethyl, ethenyl, propyl, propenyl, propargyl, butyl and amyl,
R₁ is a significance of R₂, independently of R₂, or in compounds of formula I is hydrogen,
each of R₃ to R₇, independently, is a significance of R₂, is hydrogen, methyl-X-, ethyl-X-, ethenyl-X-, propyl-X-, propenyl-X-, propargyl-X, isopropyl-X, isopropenyl-X-, t-butyl-X-, methoxymethyl-X-, methoxyethyl-X-, ethoxymethyl-X-, ethoxyethyl-X-, methoxypropyl-X- or ethoxypropyl-X-, where X is -O- or -S-.

4. A compound according to any of the preceding claims in which n = 1.

5. A compound according to one of claims 1 to 4 which is (±)-2-(3-chlorobenzyl) butanol.

6. Composition which contains at least one compound of formula I or II according to one of claims 1 to 5 and a compound selected from alcohols, surfactants and solvents.

7. Composition according to Claim 6 which contains a compound of formula I or II in a quantity of 0.01 to 10 % by wt., in particular 0.05 to 8 % by wt. and preferably 0.1 to 5 % by wt.

8. Composition according to claim 6 or 7 which contains
a) 0.01 to 10 % by wt. of a compound of formula I or II, and
b) 0.1 to 90 % by wt. of a compound selected from C₁-C₆ alkyl alcohols, unsubstituded or substituted with a C₆-C₁₂ aryl, aralkyl or aryloxy group, anionic, cationic, amphoteric or nonionic surfactants, dimethylform-amide, betaines and glycerine.

9. Composition according to any of claims 6 to 8 which is a disinfectant, antiseptic, antimycotic, deodorant or preservative.

10. Process for the production of a compound of formula I according to claim 1 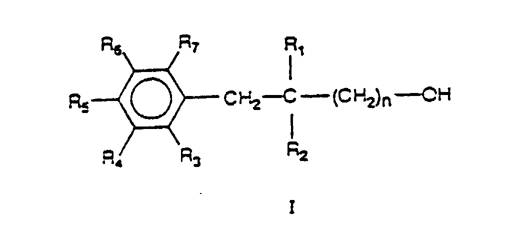 in which
R₂ is selected from C₁-C₈ alkyl, uninterrupted or interrupted by oxygen and/or sulphur atoms, C₂-C₈ alkenyl and C₃-C₈ alkynyl,
R₁ is a significance of R₂, independently of R₂, or is hydrogen,
each of R₃ to R₇, independently, is a significance of R₂, optionally attached to the aromatic ring by -S- or -O-, is H, halogen, nitrile or thiocyanate, and
n is 1 or 2,
wherein
a) a malonic acid dialkyl ester is monoalkylated, as a result of which the group R₂ is introduced,
b) the monoalkylated malonic acid alkyl ester is dialkylated with a benzyl halide optionally substituted at the aromatic ring, as a result of which the groups R₃ to R₇ are introduced, provided they are not hydrogen,
c) the dialkylated malonic acid dialkyl ester is saponified and decarboxylated, as a result of which the correspondingly 3-aryl-substituted propionic acid results and
d) this 3-aryl-substituted propionic acid is reduced with the formation of the desired alcohol of formula I.

11. Process for the production of a compound of formula II according to claim 1 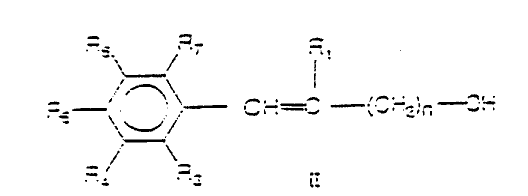 in which
R₁ is selected from C₁-C₈ alkyl, uninterrupted or interrupted by oxygen and/or sulphur atoms, C₂-C₈ alkenyl and C₃-C₈ alkynyl,
each of R₃ to R₇, independently, is a significance of R₁, optionally attached to the aromatic ring by -S- or -O-, is H, halogen, nitrile or thiocyanate, and
n is 1 or 2,
wherein in the case of n = 1 a corresponding aromatic aldehyde is condensed with an anhydride with simultaneous decarboxylation and then the resulting acid is reduced with lithium aluminium hydride, or in the case of n = 2 the tosylate of the respective alcohol with n = 1 is substituted nucleophilically by NaCN and is saponified and the resulting acid is reduced with lithium aluminium hydride to give the desired alcohol.

12. Use of a compound of formula I or II 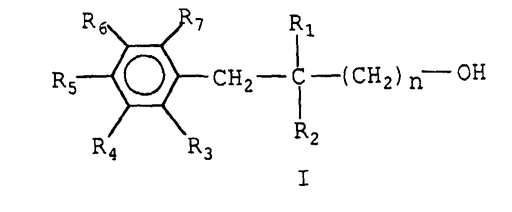 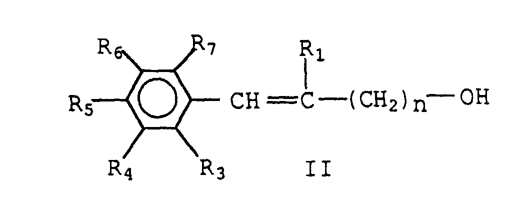 in which
R₂ is selected from C₁-C₈ alkyl, uninterrupted or interrupted by oxygen and/or sulphur atoms, C₂-C₈ alkenyl and C₃-C₈ alkynyl,
R₁ is a significance of R₂, independently of R₂, or in compounds of formula I is hydrogen,
each of R₃ to R₇, independently, is a significance of R₂, optionally attached to the aromatic ring by -S- or -O-, is H, halogen, nitrile or thiocyanate, and
n is 1 or 2,
as biocidal active ingredients,
with the proviso, that in compounds of formula I
where
R₁ and all groups R₃, R₄, R₆ and R₇ are hydrogen and
R₅ is isopropyl, tert. butyl, then n = 2.

## Patentansprüche

1. Verbindung der Formel I oder II 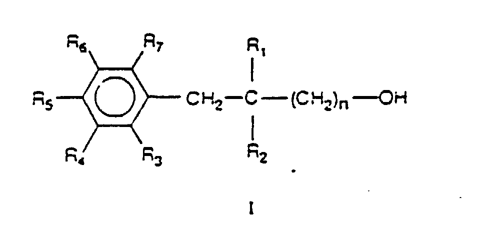 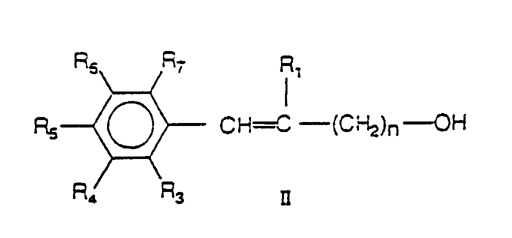 in denen
R₂ ausgewählt ist aus C₁- bis C₈-Alkyl, nicht unterbrochen oder unterbrochen durch Sauerstoff- und/oder Schwefelatome, C₂- bis C₈-Alkenyl und C₃- bis C₈-Alkinyl,
R₁ eine Bedeutung von R₂ aufweist, unabhängig von R₂, oder in Verbindungen der Formel I Wasserstoff ist,
jedes von R₃ bis R₇, unabhängig voneinander, eine Bedeutung von R₂, gegebenenfalls gebunden an den aromatischen Ring über -S- oder -O- aufweist, H, Halogen, Nitril oder Thiocyanat ist, und
n 1 oder 2 ist,
mit der Maßgabe, daß in Verbindungen der Formel I
i) wenn R₁ und alle Gruppen R₃ bis R₇ Wasserstoff sind, n = 2 ist,
ii) wenn R₁ und R₂ C₁- bis C₆-Alkyl sind und a) alle Gruppen R₃ bis R₇ Wasserstoff sind oder b) R₅ Methyl, Methoxy oder Chlorid ist und alle anderen Gruppen R₃, R₄, R₆ und R₇ Wasserstoff sind, n = 2 ist,
iii) wenn R₁, R₂ und R₄ Methyl sind und alle Gruppen R₃ und R₅ bis R₇ Wasserstoff sind, n = 2 ist,
iv) wenn R₁ und alle Gruppen R₃, R₄, R₆ und R₇ Wasserstoff sind und R₅ Methyl, Isopropyl, tert.-Butyl oder Methoxy ist, n = 2 ist,
v) wenn R₁, R₃, R₆ und R₇ Wasserstoff sind, R₂ Methyl ist und R₄ und/oder R₅ H oder C₁- bis C₆-Alkyl sind, n = 2 ist,
vi) wenn R₁ und R₄ bis R₇ Wasserstoff sind, R₂ Methyl oder Ethyl ist und R₃ Methyl oder Methoxy ist, n = 2 ist,
vii) wenn R₁, R₃, R₅ und R₇ Wasserstoff sind, R₂ Methyl ist, R₄ und R₆ Methyl sind oder R₄ Wasserstoff ist und R₆ Methyl ist, n = 2 ist
viii) wenn R₁ Wasserstoff ist, R₂ Butyl ist, R₃ und R₅ Chlorid sind und alle anderen Gruppen R₄, R₆ und R₇ Wasserstoff sind, n = 2 ist,
und mit der Maßgabe, daß in Verbindung mit der Formel II
ix) wenn R₁ C₁- bis C₅-Alkyl oder Allyl ist und alle anderen Gruppen R₃ bis R₇ Wasserstoff sind, n = 2 ist und
x) wenn R₁ Methyl ist, R₅ Methyl ist und alle anderen Gruppen R₃, R₄, R₆ und R₇ Wasserstoff sind, n = 2 ist.

2. Verbindung nach Anspruch 1, bei der
R₂ ausgewählt ist aus C₁- bis C₅-Alkyl, nicht unterbrochen oder unterbrochen durch Sauerstoff- und/oder Schwefelatome, C₂- bis C₅-Alkenyl und C₃- bis C₅-Alkinyl,
R₁ eine Bedeutung von R₂ hat, unabhängig von R₂, oder in Verbindungen der Formel I Wasserstoff ist,
jedes von R₃ bis R₇, unabhängig voneinander, eine Bedeutung von R₂, gegebenenfalls gebunden an den aromatischen Ring über -S- oder -O- aufweist, Wasserstoff, Fluor, Chlor oder Brom ist.

3. Verbindung nach Anspruch 1 oder 2, in der
R₂ Methyl, Ethyl, Ethenyl, Propyl, Propenyl, Propargyl, Butyl oder Amyl ist,
R₁ eine Bedeutung von R₂ aufweist, unabhängig von R₂, oder in Verbindungen der Formel I Wasserstoff ist,
jedes von R₃ bis R₇, jeweils unabhängig voneinander, eine Bedeutung von R₂ aufweist, Wasserstoff, Methyl-X-, Ethyl-X-, Ethenyl-X-, Propyl-X-, Propenyl-X-, Propargyl-X-, Isopropyl-X-, Isopropenyl-X-, tert.-Butyl-X-, Methoxymethyl-X-, Methoxyethyl-X-, Ethoxymethyl-X-, Ethoxyethyl-X-, Methoxypropyl-X- oder Ethoxypropyl-X- ist, wobei X -O- oder -S- ist.

4. Verbindung nach einem der vorhergehenden Ansprüche, in der n = 1 ist.

5. Verbindung nach einem der Ansprüche 1 bis 4, die (±)-2-(3-Chlorbenzyl)butanol ist.

6. Zusammensetzung, die mindestens eine Verbindung der Formel I oder II gemäß einem der Ansprüche 1 bis 5 und eine Verbindung ausgewählt aus Alkoholen, Tensiden und Lösungsmitteln enthält.

7. Zusammensetzung nach Anspruch 6, die eine Verbindung der Formel I oder II in einer Menge von 0,01 bis 10 Gew.-%, insbesondere 0,05 bis 8 Gew.-% und vorzugsweise 0,1 bis 5 Gew.-% enthält.

8. Zusammensetzung nach Anspruch 6 oder 7, die
a) 0,01 bis 10 Gew.-% einer Verbindung der Formel I oder II und
b) 0,1 bis 90 Gew.-% einer Verbindung ausgewählt aus C₁-bis C₆-Alkylalkoholen, unsubstituiert oder substituiert mit einer C₆- bis C₁₂-Aryl-, Aralkyl- oder Aryloxygruppe, anionischen, kationischen, amphoteren oder nichtionischen Tensiden, Dimethylformamid, Betainen und Glycerin enthält.

9. Zusammensetzung nach einem der Ansprüche 6 bis 8, die ein Desinfektionsmittel, Antiseptikum, Antimykotikum, Deodorant oder Konservierungsmittel ist.

10. Verfahren zur Herstellung einer Verbindung der Formel I gemäß Anspruch 1 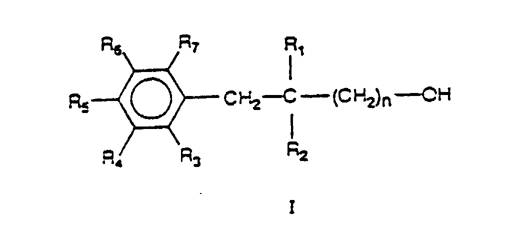 in der
R₂ ausgewählt ist aus C₁- bis C₈-Alkyl, nicht unterbrochen oder unterbrochen durch Sauerstoff- und/oder Schwefelatome, C₂- bis C₈-Alkenyl und C₃- bis C₈-Alkinyl,
R₁ eine Bedeutung von R₂ hat, unabhängig von R₂, oder Wasserstoff ist,
jedes von R₃ bis R₇, unabhängig voneinander, eine Bedeutung von R₂ hat, gegebenenfalls an den aromatischen Ring über -S-oder -O- gebunden ist, H, Halogen, Nitril oder Thiocyanat ist, und
n 1 oder 2 ist,
bei dem
a) ein Malonsäuredialkylester monoalkyliert wird, wobei als Ergebnis davon die Gruppe R₂ eingeführt wird,
b) der monoalkylierte Malonsäurealkylester mit einem Benzylhalogenid dialkyliert wird, das gegebenenfalls am aromatischen Ring substituiert ist, wobei als Ergebnis davon die Gruppen R₃ bis R₇ eingeführt werden, vorausgesetzt, daß sie nicht Wasserstoff sind,
c) der dialkylierte Malonsäuredialkylester verseift und decarboxyliert wird, wobei als Folge davon die entsprechende 3-arylsubstituierte Propionsäure hergestellt wird, und
d) diese 3-arylsubstituierte Propionsäure unter Bildung des gewünschten Alkohols der Formel I reduziert wird.

11. Verfahren für die Herstellung einer Verbindung der Formel II gemäß Anspruch 1 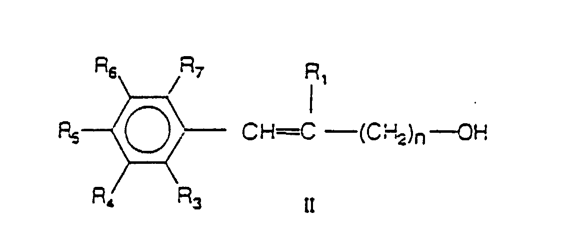 in der
R₁ ausgewählt ist aus C₁- bis C₈-Alkyl, nicht unterbrochen oder unterbrochen durch Sauerstoff- und/oder Schwefelatome, C₂- bis C₈-Alkenyl und C₃- bis C₈-Alkinyl,
jedes von R₃ bis R₇, jeweils unabhängig voneinander, eine Bedeutung von R₁ aufweist, gegebenenfalls an den aromatischen Ring über -S- oder -O- gebunden ist, H, Halogen, Nitril oder Thiocyanat ist, und
n 1 oder 2 ist,
bei dem im Fall von n = 1 ein entsprechendes aromatisches Aldehyd mit einem Anhydrid unter gleichzeitiger Decarboxylierung kondensiert wird und dann die resultierende Säure mit Lithiumaluminiumhydrid reduziert wird, oder im Fall von n = 2 das Tosylat des betreffenden Alkohols mit n = 1 nukleophil mit NaCN substituiert wird und verseift wird und die resultierende Säure mit Lithiumaluminiumhydrid unter Bildung des gewünschten Alkohols reduziert wird.

12. Verwendung einer Verbindung der Formel I oder II 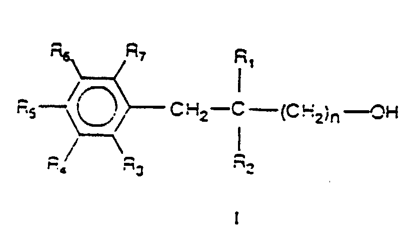 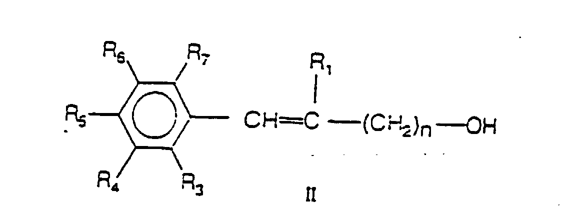 in denen
R₂ ausgewählt ist aus C₁- bis C₈-Alkyl, nicht unterbrochen oder unterbrochen durch Sauerstoff- und/oder Schwefelatome, C₂- bis C₈-Alkenyl und C₃- bis C₈-Alkinyl,
R₁ eine Bedeutung von R₂ hat, unabhängig von R₂, oder in Verbindungen der Formel I Wasserstoff ist,
jedes von R₃ bis R₇, jeweils unabhängig voneinander, eine Bedeutung von R₂ hat, gegebenenfalls an den aromatischen Ring über -S- oder -O- gebunden ist, H, Halogen, Nitril oder Thiocyanat ist, und
n 1 oder 2 ist,
als biozid wirksamer Bestandteil,
mit der Maßgabe, daß in Verbindungen der Formel I,
wenn R₁ und alle Gruppen R₃, R₄, R₆ und R₇ Wasserstoff sind und R₅ Isopropyl, tert.-Butyl ist, n = 2 ist.

## Revendications

1. Composé de la formule I ou II, 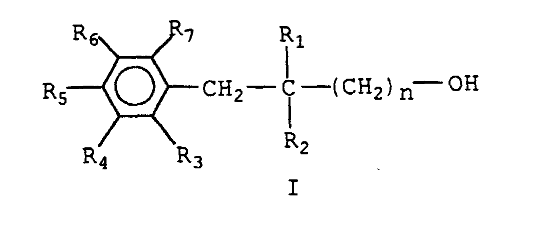 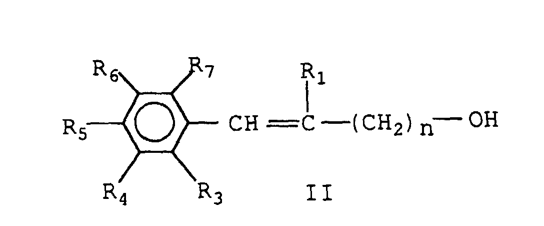 dans laquelle
R₂ est sélectionné parmi alkyle C₁-C₈, non interrompu ou interrompu par des atomes d'oxygène et/ou de soufre, alcényle C₂-C₈ et alkynyle C₃-C₈,
R₁ a l'importance de R₂, indépendamment de R₂, ou dans des composés de formule I est de l'hydrogène,
chacun de R₃ à R₇, indépendamment a l'importance de R₂, facultativement attaché au cycle aromatique par -S- ou -O-, est H, halogène, nitrile ou thiocyanate, et
n est 1 ou 2,
à condition que dans les composés de formule I
i) quand R₁ et tous les groupes R₃ à R₇ sont hydrogène, alors n = 2;
ii) quand R₁ et R₂ sont alkyle C₁-C₆ et a) tous les groupes R₃ à R₇ sont hydrogène ou b) R₅ est méthyle, méthoxy ou chlorure et tous les autres groupes R₃, R₄, R₆ et R₇ sont hydrogène, alors n=2;
iii) quand R₁, R₂ et R₄ sont méthyle et tous les groupes R₃ et R₅ à R₇ sont hydrogène, alors n=2;
iv) quand R₁ et tous les groupes R₃, R₄, R₆ et R₇ sont hydrogène et R₅ est méthyle, isopropyle, butyle tertiaire ou méthoxy, alors n = 2;
v) quand R₁, R₃, R₆ et R₇ sont hydrogène, R₂ est méthyle et R₄ et/ou R₅ sont H ou alkyle C₁-C₅, alors n = 2;
vi) quand R₁ et R₄ à R₇ sont hydrogène, R₂ est méthyle ou éthyle et R₃ est méthyle ou méthoxy, alors n = 2;
vii) quand R₁, R₃, R₅ et R₇ sont hydrogène, R₂ est méthyle, R₄ et R₆ sont méthyle ou R₄ est hydrogène et R₆ est méthyle, alors n = 2;
viii) quand R₁ est hydrogène et R₂ est butyle, R₃ et R₅ sont chlorure et tous les autres groupes R₄, R₆ et R₇ sont hydrogène, alors n = 2;
et à condition que, dans les composés de formule II
ix) quand R₁ est alkyle C₁-C₅ ou allyle et que tous les autres groupes R₃ à R₇ sont hydrogène, alors n = 2, et
x) quand R₁ est méthyle, R₅ est méthyle et tous les autres groupes R₃, R₄, R₆ et R₇ sont hydrogène, alors n = 2.

2. Un composé selon la revendication 1,
dans lequel
R₂ est sélectionné parmi alkyle C₁-C₅, non interrompu ou interrompu par des atomes d'oxygène et/ou de soufre, alcényle C₂-c₅ et alkynyle C₃-C₅.
R₁ a l'importance de R₂, indépendamment de R₂, ou dans des composés de formule I est hydrogène,
chacun de R₃ à R₇, indépendamment, a l'importance de R₂, facultativement attaché à un cycle aromatique par -S- ou -O-, est hydrogène, fluore, chlore ou brome.

3. Un composé selon la revendication 1 ou 2 dans lequel
R₂ est méthyle, éthyle, éthényle, propyle, propényle, propargyle, butyle et amyle,
R₁ a l'importance de R₂, indépendamment de R₂, ou dans des composés de formule I est hydrogène,
chacun de R₃ à R₇, indépendamment, a l'importance de R₂, est hydrogène, méthyl-X-, éthyl-X-, éthényl-X-, propyl-X-, propényl-X-, propargyl-X, isopropyl-X, isopropényl-X-, t-butyl-X-, méthoxyméthyl-X-, méthoxyéthyl-X-, éthoxyméthyl-X-, éthoxyéthyl-X-, méthoxypropyl-X- ou éthoxypropyl-X-, où X est -O- ou -S-.

4. Composé selon l'une quelconque des revendications précédentes dans lequel n = 1.

5. Composé selon l'une quelconque des revendications 1 à 4 qui est (±)-2-(3-chlorobenzyl)butanol.

6. Composition qui contient au moins un composé de formule I ou II selon l'une des revendications 1 à 5 et un composé sélectionné parmi des alcools, agents tensio-actifs et solvants.

7. Composition selon la revendication 6 qui contient un composé de formule I ou II en une quantité de 0,01 à 10% en poids, en particulier 0,05 à 8% en poids et de préférence 0,1 à 5% en poids.

8. Composition selon la revendication 6 ou 7 qui contient
a) 0,01 à 10% en poids d'un composé de formule I ou II, et
b) 0,1 à 90% en poids d'un composé sélectionné parmi des alcools alkyliques C₁-C₆, non substitués ou substitués par un groupe aryloxy, aralkyle ou aryle C₆-C₁₂, des agents tensio-actifs anioniques, cationiques, amphotères ou non ioniques, du diméthylformamide, des bétaines et de la glycérine.

9. Composition selon l'une quelconque des revendications 6 à 8 qui est un désinfectant, un antiseptique, un antimycotique, un déodorant ou un conservateur.

10. Procédé pour la production d'un composé de la formule 1 selon la revendication 1 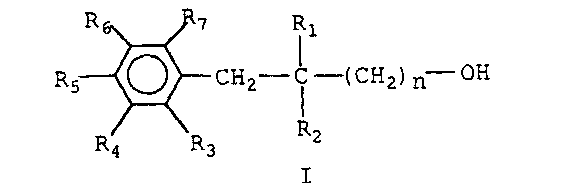 dans laquelle
R₂ est sélectionné parmi alkyle C₁-C₈, non interrompu ou interompu par des atomes d'oxygène et/ou de soufre, alcényle C₂-C₈ et alkynyle C₃-C₈,
R₁ a l'importance de R₂, indépendamment, de R₂, ou dans des composés de formule I est de l'hydrogène,
chacun de R₃ à R₇, indépendamment, a l'importance de R₂, facultativement attaché au cycle aromatique par -S- ou -O-, est H, halogène, nitrile ou thiocyanate, et
n est 1 ou 2,
où
a) un dialkyl ester de l'acide malonique est monoalkylé, par suite de quoi le groupe R₂ est introduit,
b) l'alkyl éther de l'acide malonique monoalkylé et dialkylé avec un halogénure de benzyle, facultativement substitué au cycle aromatique, par suite de quoi les groupes R₃ à R₇ sont introduits à condition qu'ils ne soient pas de l'hydrogène,
c) le dialkyl ester de l'acide malonique dialkylé est saponifié et décarboxylé, par suite de quoi il en résulte l'acide propionique 3-aryle substitué de manière correspondante et
d) cet acide propionique 3-aryle substitué est réduit avec la formation de l'alcool souhaité de formule I

11. Procédé pour la production d'un composé de formule II selon la revendication 1 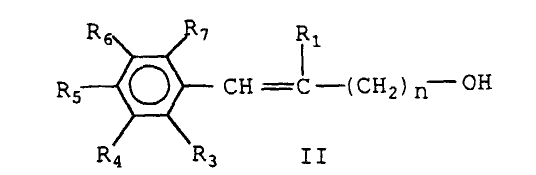 dans laquelle
R₁ est sélectionné parmi alkyle C₁-C₃, non interrompu ou interrompu par des atomes d'oxygène et/ou de soufre, alcényle C₂-C₃ et alkynyle C₃-C₈,
chacun de R₃ à R₇, indépendamment, a l'importance de R₁, facultativement attaché au cycle aromatique par -S- ou -O-, est H, halogène, nitrile ou thiocyanate, et
n est 1 ou 2,
où, dans le cas de n = 1, un aldéhyde aromatique correspondant est condensé avec un anhydride avec décarboxylation simultanée puis l'acide résultant est réduit avec de l'hydrure de lithium aluminium, ou bien dans le cas de n = 2, la tosylate de l'alcool respectif avec n = 1 est substitué nucléophilement par NaCN et saponifié et l'acide résultant est réduit avec de l'hydrure de lithium aluminium pour donner l'alcool souhaité.

12. Utilisation d'un composé de la formule I ou II 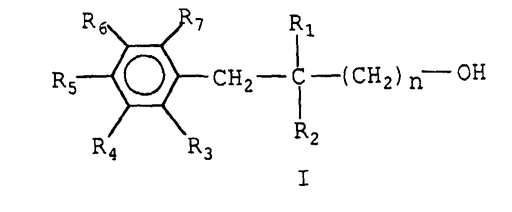 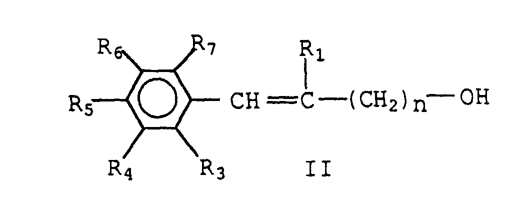 dans laquelle
R₂ est sélectionné parmi alkyle C₁-C₈, non interrompu ou interrompu par des atomes d'oxygène et/ou de soufre, alcényle C₁-C₈ et alkynyle C₃-C₈,
R₁ a l'importance de R₂, indépendamment de R₂, ou dans les composés de formule I est hydrogène,
chacun de R₃ à R₇, indépendamment, a l'importance de R₂, facultativement attaché au cycle aromatique par -S- ou -O-, est halogène, nitrile ou thiocyanate, et
n est 1 ou 2,
en tant qu'ingrédients biocides actifs,
à condition que, dans les composés de formule I quand R₁ et tous les groupes R₃, R₄, R₆ et R₇ sont hydrogène
et que R₅ est isopropyle, butyle tertiaire, alors n = 2.
